# FASCICULE DE BREVET EUROPEEN

(11) **EP 1 173 585 B1**
(45) Date de publication et mention de la délivrance du brevet: **02.07.2008**
(21) Numéro de dépôt: 00925398.0
(22) Date de dépôt: 05.05.2000
(51) Int. Cl.: C12N 15/55, C12N 9/14, C12N 15/80, C12N 1/15, C12P 7/18, C12P 7/00, C12P 7/22, C12P 41/00, C12P 13/00

(54) **EPOXYDE HYDROLASES D'ORIGINE ASPERGILLUS**
EPOXIDHYDROLASE AUS ASPERGILLUS
EPOXIDE HYDROLASES OF ASPERGILLUS ORIGIN

(30) Priorité: 05.05.1999 FR 9905711
(43) Date de publication de la demande: 23.01.2002
(73) Titulaire: Centre National de la Recherche Scientifique, 75794 Paris Cedex 16 (FR); Université de la Méditerranée, 13284 Marseille Cédex 07 (FR)
(72) Inventeur: ARAND, Michael, D-55246 Mainz-Kastheim (DE); ARCHELAS, Alain, Robert, F-13011 Marseille (FR); BARATTI, Jacques, F-13600 La Ciotat (FR); FURSTOSS, Roland, F-13009 Marseille (FR)
(74) Mandataire: Grosset-Fournier, Chantal Catherine
(86) Numéro de dépôt international: PCT/FR2000/001217
(87) Numéro de publication internationale: WO 2000/068394

(56) Documents cités:
- ARCHELAS A ET AL: "Epoxide hydrolases: new tools for the synthesis of fine organic chemicals" TRENDS IN BIOTECHNOLOGY,GB,ELSEVIER PUBLICATIONS, CAMBRIDGE, vol. 16, no. 3, 1 mars 1998 (1998-03-01), pages 108-116, XP004108588 ISSN: 0167-7799 cité dans la demande
- MOUSSOU P ET AL: "Microbiological Transformations 40. Use of Fungal Epoxide Hydrolases for the Synthesis of Enantiopure Alkyl Epoxides" TETRAHEDRON,NL,ELSEVIER SCIENCE PUBLISHERS, AMSTERDAM, vol. 54, no. 8, 19 février 1998 (1998-02-19), pages 1563-1572, XP004106670 ISSN: 0040-4020
- CLEIJ M ET AL: "Microbiological transformations. Part 42: A two-liquid-phase preparative scale process for an epoxide hydrolase catalysed resolution of para-bromo-alpha-methyl styrene oxide. Occurrence of a surprising enantioselectivity enhancement" TETRAHEDRON: ASYMMETRY,NL,ELSEVIER SCIENCE PUBLISHERS, AMSTERDAM, vol. 9, no. 11, 5 juin 1998 (1998-06-05), pages 1839-1842, XP004123469 ISSN: 0957-4166
- NELLAIAH H ET AL.: "Enantioselective hydrolysis of p-nitrostyrene oxide by an epoxide hydrolase preparation from Aspergillus niger" BIOTECHNOLOGY AND BIOENGINEERING, vol. 49, 1996, pages 70-77, XP002130510 cité dans la demande
- DATABASE EMEST3 [en ligne] EMBL, Heidelberg, Germany; AC/ID AA784929, 8 février 1998 (1998-02-08) KUPFER D ET AL.: "An Aspergillus nidulans EST database" XP002130511
- ARAND M ET AL.: "Cloning and molecular characterization of a soluble epoxide hydrolase from Aspergillus niger is related to mammalian microsomal epoxide hydrolase" BIOCHEMICAL JOURNAL, vol. 344, 15 novembre 1999 (1999-11-15), pages 273-280, XP000938551
- MORISSEAU C. ET AL ENZYME AND MICROBIAL TECHNOLOGY vol. 20, 1997, pages 446 - 452

## Description

La présente invention a pour objet des protéines d'origine fongique ou des protéines dérivées de ces dernières, ayant une activité époxyde hydrolase, ainsi que leurs utilisations, notamment pour la préparation de molécules énantiomériquement pures (ou énantiopures), tels que des époxydes et/ou des diols vicinaux à haute pureté énantiomérique.

Les époxydes ou les diols vicinaux sont des composés importants en synthèse organique. Dans le cas où ils possèdent une structure chirale, ils peuvent être utilisés soit sous forme racémique, soit sous forme optiquement enrichie ou même énantiomériquement pure. Dans le premier cas, ils constituent des produits de base pour l'industrie chimique (monomères polymérisables ou composants de produits industriels tels glycol, propylène glycol etc.). Dans le deuxième cas, ils peuvent être utilisés comme synthons chiraux pour la production de divers produits optiquement purs comme par exemple des molécules biologiquement actives commercialisées par l'industrie pharmaceutique ou phytosanitaire ou de matériaux à propriétés optiques spécifiques (cristaux liquides par exemple).

C'est pourquoi diverses stratégies de synthèse chimique ont été élaborées pour en réaliser la production. En ce qui concerne l'obtention des diols ces stratégies impliquent souvent l'hydrolyse d'un époxyde en milieu acide ou basique minéral plus ou moins concentré, ce qui génère par-là même des coûts supplémentaires dus au retraitement des eaux mères et/ou des sels formés au cours du procédé.

Dans le cas où ces molécules doivent être produites sous forme optiquement active, plusieurs stratégies ont été décrites et développées (Schurig et al. 1992, Pedragosa-Moreau et al. 1995). Par exemple, la réaction d'oxydation de Katzuki-Sharpless permet la transformation d'une oléfine en époxyde optiquement enrichi à l'aide d'un catalyseur organométallique chiral à base de titane. Cependant cette approche est limitée à des oléfines porteuses d'une fonction alcool en position alpha de la double liaison, nécessaire à la coordination du catalyseur.

D'autres méthodes ont été développées plus récemment, et sont elles aussi basées pour la plupart sur l'utilisation de catalyseurs organométalliques impliquant très souvent des métaux lourds comme le manganèse ou le cobalt. Cependant, bien que très efficaces sur certains types de substrats, elles ne présentent que des sélectivités moyennes sur d'autres familles de molécules. Dans tous les cas, elles sont difficilement utilisables au niveau industriel compte tenu des contraintes techniques engendrées par l'utilisation des métaux lourds impliqués.

Diverses méthodologies de biocatalyse ont été décrites afin de palier ce problème. Elles mettent en oeuvre des stratégies indirectes impliquant l'utilisation d'enzymes comme par exemple des lipases, des peroxydases ou des monooxygénases (Archelas et al. 1997). Cependant la plupart de ces approches nécessitent l'élaboration de systèmes coûteux de recyclage de cofacteurs, ce qui les rend là encore particulièrement difficiles et onéreuses à mettre en oeuvre à un niveau préparatif.

L'utilisation d'une enzyme permettant de réaliser l'hydrolyse directe d'un époxyde constitue donc un moyen original et intéressant de préparation directe d'époxydes optiquement enrichis ou de diols achiraux, racémiques ou optiquement enrichis. Ces enzymes, appelées Époxyde Hydrolases, présentent l'avantage - d'une part de ne pas nécessiter de cofacteur et, d'autre part, - de permettre de réaliser dans des conditions particulièrement douces l'addition d'une molécule d'eau sur un époxyde. Si le substrat est chiral, et selon l'énantiosélectivité et la régiosélectivité de cette addition, le diol obtenu sera racémique ou énantiomériquement enrichi (Archelas et al. 1998).

Bien que de nombreux travaux aient été consacrés à ce type d'enzyme présent chez les mammifères, leur utilisation en synthèse organique n'est pas envisageable compte tenu de la difficulté de les obtenir en quantité suffisante.

La possibilité d'utiliser des époxydes hydrolases d'origine microbienne (bactéries, levures, champignons) -susceptibles d'être produites en grandes quantités par simple fermentation microbienne- comme « outil » en synthèse organique constituerait donc un progrès considérable.

Des exemples de préparation de composés optiquement actifs par l'utilisation de microorganismes en tant que biocatalyseurs ont été décrits, mais ne concernent que des activités enzymatiques non caractérisées détectées dans divers microorganismes. Ainsi, dans la demande de brevet européen EP n° 611 826 (Daicel Chemical Industries Co., LTD), les exemples de microorganismes donnés, capables de produire un époxyde (S) optiquement actif à partir d'un époxyde racémique, sont notamment une souche de microorganisme appartenant au genre *Candida, Rhodosporidium, Rhodococcus* et *Nosardioides.* Les exemples de microorganismes capables de produire un époxyde (R) optiquement actif, sont notamment une souche de microorganisme appartenant au genre *Trichosporon, Geotrichum, Corynebacterium, Micrococcus,* et *Brevibacterium.*

L'oxyde de styrène est l'un des substrats test le plus utilisé dans les études conduites sur les époxyde hydrolases de mammifères, et divers dérivés substitués sur le cycle aromatique de ce substrat modèle ont aussi été étudiés dans ce contexte (Dansette et al. 1978, Westkaemper et al. 1981). Plus récemment, les études réalisées avec des activités enzymatiques d'origine microbienne ont aussi utilisé ce substrat modèle, et les Inventeurs ont eux-mêmes montré que ces molécules peuvent être hydrolysées de façon énantiosélective par une souche du champignon *Aspergillus niger,* enregistrée au Muséum d'Histoire Naturelle (Paris) sous le numéro LCP521 (Lab. de Cryptogamie, 12 rue Buffon, 75005 Paris, France) (Pedragosa-Moreau et al. 1996).

Toutefois, les expériences d'hydrolyse énantiosélective effectuées à l'aide de champignons, tel que le champignon *Aspergillus niger,* décrites jusqu'à présent, font intervenir les cellules entières ou des extraits cellulaires du champignon, ce qui pose un certain nombre de problèmes techniques de mise en oeuvre, ne donne pas de bons rendements, et ne permet pas de définir la structure du catalyseur biologique utilisé.

L'utilisation d'époxydes hydrolases d'origine fongique bien identifiées et
caractérisées permettrait de remédier à ces inconvénients, mais aucune époxyde hydrolase d'origine fongique n'a pu être isolée et purifiée jusqu'à présent, suggérant la possibilité qu'une telle enzyme n'était pas suffisamment stable pour être totalement isolée de son milieu naturel.

La présente invention résulte de la mise en évidence par les Inventeurs du fait qu'il est possible d'isoler et de purifier une époxyde hydrolase à partir de champignons. Ainsi la présente invention découle de l'identification (par purification, séquençage, clonage) de l'enzyme responsable de l'activité époxyde hydrolase des champignons, tels que ceux de l'espèce *Aspergillus.*

Un des buts de la présente invention est de fournir de nouvelles enzymes à activité époxyde hydrolase d'origine fongique.

Un autre but de la présente invention est de fournir les séquences nucléotidiques codant ces enzymes.

L'invention a également pour but de fournir des cellules hôtes transformées par les séquences nucléotidiques susmentionnées, dans lesquelles lesdites enzymes sont avantageusement surexprimées.

L'invention a aussi pour but de fournir des procédés d'obtention desdites enzymes, notamment par extraction et purification à partir de cellules de champignons, ou par mise en culture de cellules hôtes telles que décrites ci-dessus.

Un autre but de la présente invention est de fournir de nouveaux procédés de biocatalyse à l'aide des enzymes susmentionnées ou des cellules hôtes productrices desdites enzymes décrites ci-dessus, pour la synthèse de divers époxydes et/ou diols, ces procédés présentant des rendements supérieurs aux procédés utilisant des cellules entières, ou des extraits cellulaires de champignons précédemment décrits.

A ce titre, l'invention a plus particulièrement pour but de fournir des procédés d'hydrolyse d'époxydes achiraux ou chiraux présentant l'avantage de pouvoir être effectués dans des conditions expérimentales particulièrement douces, à savoir sans mettre en oeuvre de réactif acide ou basique minéral ou organique, notamment en milieu aqueux tamponné ou non tamponné et/ou en présence de solvants organiques miscibles ou non miscibles à l'eau. Selon les propriétés stéréochimiques intrinsèques de l'époxyde de départ, ces procédés conduisent à l'obtention d'un diol achiral, racémique ou optiquement enrichi ainsi que, si l'époxyde de départ est chiral, à la production de l'un de ses deux énantiomères sous forme optiquement enrichie, voir énantiomériquement pure.

L'invention a pour objet une protéine d'origine fongique ayant une activité époxyde hydrolase, telle qu'obtenue sous forme essentiellement pure par extraction à partir de cellules de champignons, ou par mise en culture de cellules hôtes transformées par une séquence nucléotidique codant pour la protéine fongique susmentionnée telle que définie dans les revendications.

L'activité époxyde hydrolase susmentionnée peut être mesurée en utilisant l'oxyde de *para*-nitrostyrène (pNSO) comme substrat, et en mesurant la quantité de diol formé, notamment selon la méthode suivante :
50 µL de la préparation contenant l'enzyme sont ajoutés à 410 µL du tampon phosphate de sodium 0,1 M pH 7,0 (tampon B) et le mélange est pré-incubé à 35°C pendant 2 min. Ensuite, on ajoute 40 µL d'une solution 50 mM de pNSO racémique dans le DMF (concentration finale de pNSO : 4 mM).

Après 10 min d'incubation, la réaction est stoppée par addition de 1 mL de dichlorométhane. Le mélange est agité vigoureusement afin d'extraire à la fois le substrat et le diol produit. La quantité de diol formée est déterminée après séparation sur colonne de silice par HPLC (chromatographie liquide haute pression) (Waters Associates, USA) tel que décrit précédemment (Nellaiah et al., 1996).

Une Unité époxyde hydrolase représente la quantité d'enzyme qui catalyse la formation d'une µmole de diol par minute dans les conditions ci-dessus. Après incubation avec des extraits bruts, la quantité de diol formée augmente linéairement avec le temps pendant au moins 30 min, et la vitesse de la réaction est proportionnelle à la concentration de l'enzyme dans la gamme de 0,01 à 1,2 unités (Nellaiah et al., 1996).

L'invention a plus particulièrement pour objet toute protéine telle que décrite ci-dessus, caractérisée en ce qu'elle comprend :
- la séquence SEQ ID NO : 2,
- ou tout fragment de la séquence SEQ ID NO : 2 et possédant une activité époxyde hydrolase, ledit fragment étant constitué d'au moins 10 acides aminés contigus dans la région délimitée par les acides aminés situés aux positions 1 et 339 de la séquence SEQ ID NO : 2.

L'invention concerne plus particulièrement toute protéine décrite ci-dessus, caractérisée en ce qu'elle correspond à une époxyde hydrolase fongique sous forme essentiellement pure, telle qu'obtenue par extraction et purification à partir de cultures de cellules de champignons de l'espèce *Aspergillus.*

A ce titre l'invention a plus particulièrement pour objet toute protéine susmentionnée, caractérisée en ce qu'elle correspond à l'époxyde hydrolase fongique sous forme essentiellement pure représentée par SEQ ID NO : 2, telle qu'obtenue par extraction et purification à partir de cultures de cellules de souches *d'Aspergillus niger* ou *d'Aspergillus turingensis.*

L'invention concerne également toute protéine telle que décrite ci-dessus, caractérisée en ce qu'elle correspond à une époxyde hydrolase fongique recombinante, telle qu'obtenue sous forme essentiellement pure par transformation de cellules hôtes appropriées à l'aide de vecteurs contenant :
- la séquence nucléotidique SEQ ID NO : 1 codant l'époxyde hydrolase représentée par SEQ ID NO : 2, ou toute séquence dérivée de SEQ ID NO : 1 par dégénérescence du code génétique, et codant l'époxyde hydrolase représentée par SEQ ID NO : 2,
- ou toute séquence dérivée de la séquence SEQ ID NO : 1, notamment par substitution, suppression ou addition d'un ou plusieurs nucléotides, et codant une enzyme possédant une activité époxyde hydrolase, ladite séquence dérivée ayant de préférence une homologie d'au moins environ 45 % , notamment supérieure à environ 80 %, avec la séquence SEQ ID NO : 1,
- ou tout fragment de la séquence SEQ ID NO : 1, ou d'une séquence dérivée de cette dernière telle que définie ci-dessus, et codant une enzyme possédant une activité époxyde hydrolase, ledit fragment étant de préférence constitué d'au moins environ 20 nucléotides contigus dans la région délimitée par les nucléotides situés aux positions 1 et 1197 de la séquence SEQ ID NO : 1.

A ce titre, l'invention a plus particulièrement pour objet l'époxyde hydrolase fongique recombinante représentée par SEQ ID NO : 2, telle qu'obtenue par transformation de cellules hôtes appropriées à l'aide de vecteurs contenant la séquence nucléotidique SEQ ID NO : 1, ou toute séquence dérivée de SEQ ID NO : 1 par dégénérescence du code génétique, et codant l'époxyde hydrolase représentée par SEQ ID NO : 2.

L'invention concerne également toute séquence nucléotidique codant une protéine d'origine fongique à activité époxyde hydrolase telle que définie ci-dessus.

L'invention a plus particulièrement pour objet toute séquence nucléotidique susmentionnée, caractérisée en ce qu'elle comprend :
- la séquence représentée par SEQ ID NO : 1 codant l'époxyde hydrolase représentée par SEQ ID NO : 2,
- ou toute séquence dérivée de la séquence SEQ ID NO : 1 par dégénérescence du code génétique, et codant l'époxyde hydrolase représentée par SEQ ID NO : 2,
- ou toute séquence dérivée de la séquence SEQ ID NO : 1, notamment par substitution, suppression ou addition d'un ou plusieurs nucléotides, et codant une enzyme possédant une activité époxyde hydrolase, ladite séquence dérivée ayant de préférence une homologie d'au moins environ 45 %, notamment supérieure à environ 80 %, avec la séquence SEQ ID NO : 1,
- ou tout fragment de la séquence SEQ ID NO : 1, ou d'une séquence dérivée de cette dernière telle que définie ci-dessus, et codant une enzyme possédant une activité époxyde hydrolase, ledit fragment étant de préférence constitué d'au moins environ 20 nucléotides contigus dans la région délimitée par les nucléotides situés aux positions 1 et 1197 de la séquence SEQ ID NO : 1,
- ou toute séquence nucléotidique complémentaire des séquences ou fragments susmentionnés,
- ou toute séquence nucléotidique codant une enzyme possédant une activité époxyde hydrolase, et susceptible d'hybrider avec une des séquences ou fragments susmentionnés,
les séquences ou fragments susmentionnés étant sous forme simple brin ou double brin.

L'invention concerne également tout vecteur, notamment plasmide, contenant une séquence nucléotidique telle que définie ci-dessus.

Avantageusement, les séquences nucléotidiques de l'invention dans les vecteurs susmentionnés, sont placées sous le contrôle d'éléments de régulation de l'expression des protéines à activité époxyde hydrolase définies ci-dessus, notamment d'un promoteur, le cas échéant inductible, et un terminateur de transcription.

De préférence, le promoteur susmentionné est choisi parmi ceux permettant une surexpression desdites protéines dans des cellules hôtes transformées à l'aide des vecteurs, lesdites cellules hôtes étant elles-mêmes choisies parmi celles capables de surexprimer lesdites protéines, notamment parmi les bactéries, les virus, les levures, les champignons, les plantes ou les cellules de mammifères.

L'invention concerne également toute cellule hôte, choisie notamment parmi les bactéries, les virus, les levures, les champignons, les plantes ou les cellules de mammifères, ladite cellule hôte étant transformée, notamment à l'aide d'un vecteur tel que défini ci-dessus, de manière à ce que son génome contienne contenant une séquence nucléotidique susmentionnée codant une protéine à activité époxyde hydrolase.

L'invention a également pour objet l'utilisation de protéines à activité époxyde hydrolase telles que définies ci-dessus, en tant que biocatalyseurs enzymatiques dans le cadre de la mise en oeuvre de procédés de préparation d'époxydes ou de diols vicinaux énantiomériquement purs, notamment dans le domaine pharmaceutique, phytosanitaire, ou dans le cadre de la fabrication de matériaux optiques spécifiques.

A ce titre, l'invention a plus particulièrement pour objet un procédé de préparation d'époxydes et/ou de diols énantiomériquement purs respectivement de formules (II) et (III) suivantes 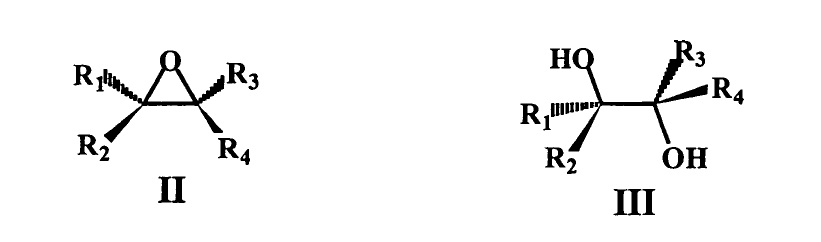 dans lesquelles R₁, R₂, R₃ et R₄ représentent des groupes quelconques, notamment des groupes caractéristiques des composés pharmaceutiques, phytosanitaires, ou des matériaux optiques spécifiques correspondant auxdits époxydes ou diols vicinaux,
ledit procédé comprenant une étape de traitement d'un mélange d'époxydes diastéréoisomères, ou d'un époxyde chiral sous forme racémique, ou d'un époxyde prochiral de formule (I) suivante : 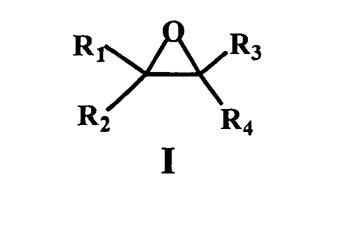 avec une protéine à activité époxyde hydrolase telle que définie ci-dessus, ou avec des cellules hôtes susmentionnées exprimant ou surexprimant une protéine à activité époxyde hydrolase telle que définie ci-dessus, ce qui conduit à l'obtention :
- d'un mélange des composés de formule (II) et (III) susmentionnés, lesdits composés de formule (II) et (III) pouvant, le cas échéant, être séparés par une étape supplémentaire de purification,
- ou du seul composé de formule (III) susmentionnée.

Dans le cas de l'obtention du seul composé de formule (III) susmentionnée, celle-ci peut se faire par un traitement concomitant ou subséquent au traitement décrit ci-dessus, notamment avec un autre réactif chimique ou enzymatique en fonction de l'époxyde de départ, par exemple avec de l'acide sulfurique, notamment dans le cas de l'oxyde de *para*-nitrostyrène (Pedragosa-Moreau et al., 1997), ou avec des cellules du champignon *Beauveria sulfurescens,* notamment dans le cas de l'oxyde de styrène (Pedragosa-Moreau et al., 1993).

Avantageusement, lorsque le procédé tel que décrit ci-dessus selon l'invention, est effectué à l'aide d'une protéine à activité époxyde hydrolase telle que définie ci-dessus, cette dernière peut être immobilisée sur un support solide tel que par exemple le DEAE cellulose ou le DEAE Sepharose, ou tout autre support ou technique permettant d'immobiliser cette enzyme.

L'invention a également plus particulièrement pour objet l'utilisation d'une protéine à activité époxyde hydrolase telle que définie ci-dessus, sous différentes formes, incluant des cellules hôtes transformées telles que décrites ci-dessus, ou des cellules entières de champignons, tel que *Aspergillus niger,* produisant cette enzyme, ou des extraits enzymatiques solubles ou lyophilisés desdites cellules, ou l'enzyme immobilisée sur un support solide tel que défini ci-dessus, dans le cadre de la mise en oeuvre d'un procédé tel que décrit ci-dessus d'hydrolyse d'un époxyde achiral.

L'invention concerne également un procédé de préparation de protéine à activité époxyde hydrolase recombinante telle que définie ci-dessus, caractérisé en ce qu'il comprend une étape de transformation de cellules hôtes, de préférence choisies parmi les bactéries, les virus, les levures, les champignons, les plantes ou les cellules de mammifères, avec un vecteur tel que décrit ci-dessus, et une étape de purification de l'époxyde hydrolase recombinante produite par lesdites cellules.

L'invention a également pour objet un procédé de préparation d'une époxyde hydrolase fongique sous forme essentiellement pure, ledit procédé comprenant :
- une étape d'extraction de l'enzyme à partir de cultures cellulaires de champignons, tels que les champignons de l'espèce *Aspergillus,* notamment par cassage du champignon à l'aide d'une presse de French ou de tout autre moyen approprié, suivie d'une étape de centrifugation à faible vitesse (environ 10 000 g), récupération du surnageant, et concentration par ultrafiltration,
- une étape de purification de l'enzyme à partir de l'extrait obtenu à l'étape précédente, notamment par passages successifs sur des colonnes de DEAE-Sepharose, Phényl-Sepharose, Mono Q et Superose 12.

L'invention sera davantage illustrée à l'aide de la description détaillée qui suit de la purification de l'époxyde hydrolase d'une souche du champignon *Aspergillus niger,* ainsi que du clonage du gène codant cette époxyde hydrolase, et d'exemples d'application de mise en oeuvre d'un procédé selon l'invention.

### A) Purification et caractérisation d'une époxyde hydrolase d'Aspergillus niger à haute énantiosélectivité

### I) Matériels et Méthodes

### 1) Réactifs

Le substrat test utilisé est l'oxyde de p-nitrostyrène racémique (pNSO). Il est synthétisé à partir de ω-bromo-4-nitro acétophénone selon une méthode décrite par Westkaemper et Hanzlik, 1980. Ses énantiomères purs (R) et (S) sont obtenus à partir de ce substrat racémique par une étape de biotransformation (Pedragosa-Moreau et al., 1996). Le diéthylaminoéthyl (DEAE)-Sepharose, le phényl-Sepharose, les colonnes "Mono Q" et Superose 12 proviennent de chez Pharmacia LKB (Uppsala, Suède). H₂ [¹⁸O] provient de chez Isotec (Miamisburg, USA) et présente une teneur de 95 % de [¹⁸O]. Toutes les chromatographies de protéines sont réalisées à l'aide du système FPLC Pharmacia à 4°C.

### 2) Organismes, conditions de croissance et préparation d'extraits

La souche du champignon *Aspergillus niger* utilisée dans cette étude est enregistrée au Museum d'Histoire Naturelle (Paris) sous le numéro LCP521 (Lab. de Cryptogamie, 12 rue Buffon, 75005 Paris, France). La culture est effectuée dans un fermenteur d'une capacité de 5L (volume liquide) dans les conditions décrite par Nellaiah et al., 1996. Les cellules sont récoltées après 40 h de culture par filtration. Elles sont suspendues dans un tampon Tris-HCl 10 mM de pH 7,1 (tampon A) contenant de la cystéine 1 mM, de l'EDTA 1 mM et du chlorure de phénylméthane sulfonyle (PMSF) 0,3 mM. Un extrait acellulaire est préparé par rupture du champignon en utilisant une presse de French, ou tout autre moyen utilisable par l'homme de l'art et une centrifugation à faible vitesse (1000 g) dans les conditions décrites par Nellaiah et al., 1996. Cet extrait est concentré à 100 mL par filtration à flux tangentiel avec une membrane ayant un seuil de coupure de 10, 40 ou jusqu'à 100 kDa. Toutes les autres manipulations sont conduites à une température de 4°C dans une solution tampon contenant de la cystéine 1 mM, de l'EDTA 1 mM et du PMSF 0,3 mM afin de prévenir l'inactivation de l'enzyme. La concentration de la protéine est déterminée par la méthode de Lowry et al., 1951, utilisant de l'albumine de sérum de boeuf comme témoin.

### 3) Purification de l'époxyde hydrolase

La solution concentrée contenant l'enzyme est déposée sur une colonne de DEAE (diéthylaminoéthyl)-Sepharose (2,5 cm x 30 cm) préalablement équilibrée avec le tampon A contenant du KCI 0,13 M. La colonne est lavée avec 360 mL de tampon d'équilibrage, et l'élution est conduite avec un gradient linéaire de KCl 0,13-0,23 M dans le tampon A (volume total : 510 mL, débit : 3 mL/min., volumes des fractions : 6 mL).

L'activité est éluée pour une concentration en chlorure de potassium de 0,17-0,20 M. Les fractions actives sont regroupées et concentrées à 5 mL par ultrafiltration. Le concentré est déposé sur une colonne de phényl-sepharose (1 cm x 10 cm), préalablement équilibrée avec le tampon A contenant (NH₄)₂SO₄ 0,25 M et 21 % (v/v) d'éthylène glycol. La colonne est lavée avec 10 mL du même tampon, et l'élution est conduite avec un gradient linéaire d'éthylène glycol 21-56 % (v/v) dans le tampon A contenant du (NH₄)₂SO₄ 0,25 M (volume total : 95 mL, débit : 0,5 mL/min., volumes des fractions : 1 mL).

L'activité est éluée avec une concentration d'éthylène glycol de 30-43 % (v/v). Les fractions actives sont regroupées et concentrées à 5 mL. Le concentré est déposé sur une colonne Mono Q (0,5 cm x 5 cm), préalablement équilibrée avec le tampon Tris-HCl 10 mM pH : 6,5 contenant du KCl 0,13 M. La colonne est lavée avec 5 mL de tampon, et l'élution est conduite avec un gradient linéaire de chlorure de potassium 0,13-0,25 M (volume total : 85 mL, débit : 0,5 mL/min., volumes des fractions : 1 mL). L'activité est éluée à une concentration de 0,15-0,16 M de chlorure de potassium. Les fractions actives sont regroupées et concentrées à 1 mL. La solution contenant l'enzyme (200 mL) est déposée sur une colonne de Superose 12 (1 cm x 30 cm) et équilibrée avec un tampon A (débit : 0,3 mL/min., volumes des fractions : 0,6 mL). Cette étape est effectuée 5 fois (200 µL chaque fois) et toutes les fractions actives sont regroupées. La préparation ainsi obtenue est conservée à 4°C.

### 4) Etude enzymatique

Les incubations avec H₂[¹⁸O] sont conduites dans des fioles de 1 mL contenant 180 µL de H₂[¹⁸O] tampon B, 20 µL de l'époxyde hydrolase purifiée et 20 µL de substrat (50 mM dans l'acétonitrile). Après 1,5 h d'incubation à 25°C sous agitation magnétique (500 rpm) le substrat restant et le produit formé sont extraits avec 2 mL de dichlorométhane. Le diol est purifié par chromatographie analytique sur silice (éluant diéthyléther). On analyse 2 µL d'échantillons par chromatographie gazeuse/spectrométrie de masse (GC/MS). Le diol restant est transformé en l'acétonide correspondant par réaction avec du 2,2 diméthyl-propane en présence de l'acide p-toluène sulfonique. L'acétonide est analysé par GC/MS tel que déjà décrit par Audier et al.,1968.

Une réaction dans un volume total de 5 mL est effectuée à 25°C, avec un substrat d'une concentration de 4,3 mM avec du DMSO (20% en volume) comme co-solvant dans un tampon phosphate de sodium 0,1 M pH 8.0.

La réaction est démarrée par addition de 13 U/L de l'enzyme purifiée. Des échantillons sont retirés toutes les 30 minutes pour la quantification des concentrations du substrat et de produit par HPLC en utilisant une colonne en phase inverse (Nellaiah et al., 1996) et pour la quantification de l'excès énantiomérique de l'époxyde et du diol par chromatographie en phase gazeuse (Nellaiah et al., 1996).

### 5) Electrophorèse sur gel de polyacrylamide

Une électrophorèse SDS-PAGE est effectuée sur une plaque de 1 mm d'épaisseur contenant un gel de résolution (10 % d'acrylamide) et un gel de concentration (4 % d'acrylamide) à pH 8,8 en présence de 0,1 % de dodecyl sulfate de sodium (SDS) (Laemmli, 1970). Les échantillons sont dissous dans un tampon Tris-HCl (62,5 mM, pH 8,8) contenant 1 % (p/v) de SDS, 10 % (v/v) de glycérol et 2 % (v/v) de β-mercaptoéthanol, et sont chauffés à 100°C pendant 2 minutes. Les protéines sont colorées avec 0,1 % (p/v) de bleu de Coomassie. Des migrations sur gels PAGE non dénaturants sont effectuées de la même façon excepté qu'il n'y a pas eu de β-mercaptoéthanol rajouté dans le tampon de résolution, et que les échantillons n'ont pas été chauffés. L'électrofocalisation est réalisée avec un gradient de pH de 3-9 à l'aide du système Pharmacia LKB "Phastsystem" et des procédures standards Pharmacia. Les protéines sont colorées avec du nitrate d'argent.

### 6) Détermination de la masse moléculaire

La masse moléculaire a été estimée après SDS-PAGE par comparaison de la mobilité (Rf) de l'époxyde hydrolase (EH) purifiée avec celle des protéines témoins suivantes : phosphorylase B (97,4 kDa), sérum albumine bovine (66,2 kDa), ovalbumine (45 kDa), anhydrase carbonique (31 kDa), inhibiteur de la trypsine (21,5 kDa) et lysozyme (14,4 kDa). La masse moléculaire de l'enzyme native est estimée à partir du profil d'élution de Superose 12 par comparaison du Kav de l'EH purifiée avec celui des protéines standard suivantes: alcool déhydrogénase (150 kDa), sérum albumine bovine (67 kDa), ovalbumine (43 kDa), chymotrypsinogène A (25 kDa) et ribonucléase A (13,7 kDa). Le volume d'exclusion et le volume mort sont déterminés en utilisant du bleu dextranne et de la vitamine B12.

### 7) Séquence d'acides aminés

Pour les analyses d'acides aminés et les déterminations de séquence N-terminale, les peptides sont transférés des gels SDS sur une membrane "glassy-bond" (Biometra, Allemagne) en utilisant des procédures standard Biorad (Hercules, USA). La composition en acides aminés de l'enzyme est déterminée après hydrolyse acide (HCl 6N à 100°C sous vide pendant 24 h) en utilisant un analyseur automatique d'acides aminés (system Beckman 6300, Allemagne). La masse moléculaire est estimée à partir de la composition en acides aminés en utilisant la méthode de Delaage (1968).

### 8) Séquences des peptides

Les protéines sont dissoutes dans un tampon SDS et séparées par SDS-PAGE. Une partie du gel est colorée avec du Bleu de Coomassie, et la bande d'intérêt est séparée à partir de l'autre partie du gel. La bande est lavée pendant 1 h avec H₂O, H₂O-CH₃OH (90 : 10), H₂O-CH₃CN (80 : 20), et H₂O-CH₃CN (50 : 50). La bande de gel est ensuite coupée en petits morceaux et séchée sous vide dans un Speed-Vac (Savant). Ensuite, 400 µL d'une solution contenant Tris-HCl 25 mM (pH 8,5), EDTA 1 mM, 0,05 % SDS, et 5 µg de la protéase Lys-c (Boehringer Mannheim) sont ajoutés, et le mélange est incubé toute la nuit à 37°C. L'hydrolysat est injecté dans une colonne HPLC en phase inverse (Vydac C₁₈ ; 2,1 x 250 mm). La colonne est éluée à un débit de 0,2 mL/min avec un gradient linéaire de 0 à 35 % de la solution B (CH₃CN, contenant 0.07 % d'acide trifluoroacétique) pendant 150 min (la solution A est constituée d'eau et de 0,07 % d'acide trifluoroacétique) et les pics sont recueillis et directement séquencés avec un microséquenceur Applied Biosystems modèle 477A.

### 9) Réaction PCR, clonage et séquençage

Les réactions PCR sont réalisées en utilisant comme amorce des oligomères dégénérés obtenus à partir des séquences partielles d'acides aminés et en utilisant de l'ADN génomique *d'Aspergillus niger* en tant que support. L'ADN génomique est extrait de 1,5 g du mycélium lavé avec de l'eau, broyé dans de l'azote liquide et suspendu dans du tampon Tris-HCl 50 mM pH 7,5, EDTA 50 mM, SDS 3 %, β-mercaptoéthanol 1 %. Après réaction pendant 1 h à 65 °C, la solution est extraite avec un mélange phénol/chloroforme/alcool isoamylique (24/24/1, v/v/v) et chloroforme/alcool isoamylique (24/1), précipitée à l'isopropanol, et le culot est dissous dans du tampon TE (Tris-HCl 10 mM pH 7,5 et EDTA 1 mM). On ajoute de la RNase (30 µg/mL) pendant 1 h à 37 °C, l'ADN est précipité à l'isopropanol, lavé dans de l'éthanol 70 % et dissous dans de l'eau. Les réactions de PCR sont conduites dans un volume total de 50 µL, en utilisant 100 ng d'ADN, dNTP 200 µM, chaque amorce à 2 µM et 2 unités de Taq polymerase (Perkin Elmer). Les réactions PCR sont effectuées par chauffage à 95°C pendant 5 min. puis réalisées pendant 30 cycles d'amplification à trois températures (1 min. à 95°C, 1 min. à 58°C, et 1 min. à 72°C). Les fragments amplifiés sont clonés dans le site ECOR V de pBluescript II SK(-) (Statagene) après traitement avec une unité/µL de transférase terminale (Boehringer). Le séquençage des fragments est réalisé à l'aide d'un kit de séquençage T7 de Pharmacia.

### II) Résultats

L'EH *d'Aspergillus niger* est purifiée à homogénéité électrophorétique en utilisant une procédure chromatographique à 4 étapes. Au total, 120 µg de l'enzyme purifiée sont préparés à partir de 24 g de mycélium sec, c'est-à-dire de 5 L de milieu de culture. Ces valeurs relativement faibles sont dues à 2 raisons :
1) le rendement d'ensemble (total) est faible (4 %) à cause de l'instabilité de l'enzyme durant la procédure de purification principalement dans les étapes de concentration par ultrafiltration lorsque la concentration de la protéine est faible ;
2) la teneur initiale de l'EH dans l'extrait cellulaire *d'Aspergillus niger* est faible: une valeur de 0,4 % des protéines solubles est calculée en utilisant l'activité spécifique de l'enzyme purifiée. Cependant, l'enzyme purifiée est responsable de toute l'activité du champignon sur pNSO. Ainsi, il n'y a probablement qu'une seule protéine active sur ce substrat dans *Aspergillus niger.*

L'époxyde hydrolase (EH) purifiée présente une seule bande en gel PAGE natif ou SDS après coloration avec le bleu de Coomassie. Les déterminations d'activité de tranches de gel obtenues après électrophorèse d'un gel de polyacrylamide non dénaturant révèlent une seule bande se situant au même niveau de la bande de la protéine marquée. Le point isoélectrique de la protéine est de 4,5 après détermination par électrofocalisation en utilisant un gradient de pH de 3 à 9 et une coloration au nitrate d'argent.

L'EH *d'Aspergillus niger* est un tétramère composé de 4 sous unités identiques de 45 kDa. Les EHs d'autres sources sont généralement des protéines monomériques ou dimériques. Cependant, l'époxyde hydrolase de *Corynebacterium* sp a récemment été décrite en tant que dodécamère (Misawa et al., 1998).

L'effet sur l'activité de plusieurs réactifs sélectifs a été testé. L'EDTA et le PMSF ne montrent aucun effet. Des agents oxydants tels que l'acide *méta-*chloroperbenzoïque ou le peroxyde d'hydrogène inhibent fortement l'activité de l'enzyme. Au contraire, des agents de réduction tels que le β-mercaptoéthanol ou la cystéine montrent un effet positif sur l'activité de l'enzyme. De plus, une forte inactivation est observée avec des agents bloquants des thiols tels que HgCl₂, 4-hydroxy-mercuribenzoate, iodoacétamide ou dithionitrobenzène (DTNB). Tous ces résultats démontrent le rôle essentiel d'un ou plusieurs résidu(s) de cystéine sur l'activité de l'époxyde hydrolase. Un effet similaire est observé avec les EH solubles (sEH) de mammifères (Wixtrom et al., 1985) et avec l'EH de *Pseudomonas* sp (Rink et al., 1997) alors que les EH microsomales (mEH) de mammifères (Wixtrom et al., 1985) ne sont pas sensibles aux réactifs de thiol.

Le profil d'activité pH et l'inhibition par l'acétophénone ω-bromo-4-nitro suggère la participation d'un résidu histidine dans le mécanisme catalytique. De plus, certains résidus cystéine sont importants pour l'activité de l'enzyme tel que démontré pour sEH de mammifères mais pas pour mEH (Wixtrom et al., 1985). Le mécanisme catalytique des sEH et mEH de mammifères pour l'hydrolyse des époxydes a récemment été élucidé (Beetham et al., 1995 ; Arand et al., 1996). Un mécanisme en 2 étapes impliquant la formation d'un ester covalent intermédiaire a été démontré avec la participation de 2 acides aspartiques et un résidu histidine. Cependant, on ne connaît pas grand chose du mécanisme catalytique des EHs microbiennes. Récemment, un mécanisme similaire a été démontré pour l'époxyde hydrolase de la bactérie *Agrobacterium radiobacter* (Rink et al., 1997). Ces éléments suggèrent que l'EH *d'Aspergillus niger* utilise un mécanisme similaire pour l'hydratation des époxydes que les EHs de mammifères. Ce mécanisme est en accord avec le procédé général de catalyse démontré pour l'hydrolyse de l'oxyde de styrène para-substitué par un extrait brut *d'Aspergillus niger* (Pedragosa-Moreau et al., 1996).

Avec le pNSO, l'addition d'un solvant organique est requise pour la solubilisation du substrat. En effet, en l'absence de co-solvant, aucune activité ne peut être détectée. Il a été montré pour d'autres EHs solubles, qu'elles n'étaient pas actives sur des substrats micellaires (Hammock et al., 1997). Ainsi, l'effet de différents co-solvants sur l'activité de l'époxyde hydrolase *d'Aspergillus niger* a été étudié. La nature des co-solvants influence grandement le rendement d'ouverture de l'époxyde, les plus fortes activités étant obtenues pour le DMF et l'acétone. La faible activité obtenue avec le THF pourrait être corrélée à l'inactivation de l'enzyme par les traces de peroxydes qui sont habituellement présentes dans le solvant.

L'enzyme est active à un pH allant de 5 à 9 avec un pic maximum à pH 7. L'enzyme est active à une température allant de 2 à 45°C avec une activité maximum à 40°C. De 2 à 40°C l'activité augmente légèrement (seulement 4 fois) tel qu'indiqué par la faible énergie d'activation (27 kJ.mol⁻¹. ⁰K⁻¹).

D'un point de vue pratique, l'EH *d'Aspergillus niger* est très intéressante pour la synthèse organique pour sa capacité à hydrolyser des époxydes racémiques de façon hautement énantiosélective. L'énantiosélectivité est due à une plus forte affinité et une constante catalytique supérieure pour l'énantiomère (R) de pNSO par rapport à l'énantiomère (S).

Le rapport des constantes spécifiques (k_{cat}/Km) indique que la vitesse initiale d'hydrolyse de l'énantiomère (R) est 55 fois plus rapide que celui de l'énantiomère (S) en partant du racémique pNSO. Ce résultat est similaire à celui obtenu avec les cellules entières sur le même substrat (Pedragosa-Moreau, 1997). De plus, la régiosélectivité de la réaction est très élevée: 97 % pour le carbone 2, tel que montré avec le champignon entier (Pedragosa-Moreau, 1996). L'énantio- et la régiosélectivité de l'hydrolyse de pNSO par l'EH purifiée *d'Aspergillus niger* sont très similaires à celles déterminées avec l'ensemble des cellules. Par conséquent, l'enzyme purifiée est responsable de toute l'activité du champignon sur pNSO.

### B) Clonage et caractérisation de l'époxyde hydrolase soluble d'Aspergillus niger qui est apparentée aux époxydes hydrolase microsomales de mammifère

### I) Procédure expérimentale

### 1) Isolement d'acides nucléiques d'Aspergillus niger (A. niger)

*Aspergillus niger* (souche n° LCP 521 susmentionnée) a été cultivée dans un milieu contenant 10 g de glucose et 20 g de liqueur de maïs (Sigma, St Louis, Cat. n° C4648) par litre de culture. L'incubation est réalisée dans un volume de 100 ml en fiole agitée à 28°C pendant 3 jours après inoculation avec des spores du champignon. Le mycélium est récolté par filtration sur tissu et gardé à -70 °C après détermination du poids humide. L'extraction d'ARN est réalisée par la méthode de Chomczynski et Sacchi (1986) en utilisant 10 mL de solution dénaturante par gramme de mycélium. Le rendement typique est de 300 µg d'ARN total par gramme de mycélium. Pour l'isolement de l'ARN, 2 g de mycélium sont homogénéisés avec un homogénéiseur en verre de type Potter dans 15 mL d'une solution de lyse (solution de chlorhydrate de guanidine 6 M, contenant 0,1 M d'acétate de sodium, pH 5,5). Après centrifugation à 10,000 g pendant 10 min., le surnageant est transféré dans un autre tube et 2,5 volumes d'éthanol sont ajoutés Les acides nucléiques précipités sont recueillis par centrifugation à 10,000 g pendant 10 min. et le culot résultant est dissous toute la nuit dans 10 mL de tampon de lyse après un bref séchage. La fraction insoluble est retirée par centrifugation et les acides nucléiques sont à nouveau précipités par addition de 25 mL d'éthanol. Le culot de centrifugation est lavé avec de l'éthanol 70 %, séché à l'air pendant 30 min. et dissous dans un tampon TE, pH 8.0.

### 2) Clonage du gène de l'EH d'Aspergillus et de l'ADNc via la technique de l'amplification en chaîne par polymérase (polymérase chain reaction : PCR)

La PCR inverse pour l'amplification du gène de l'EH *Aspergillus* a été réalisée selon le schéma suivant : 500 ng d'ADN génomique sont digérés avec une enzyme de restriction appropriée (la plupart des résultats réussis sont obtenus avec BamHI ou Cfol) et sont récupérés par une précipitation à l'éthanol après une extraction au mélange phénol/chloroforme. Sur ces 500 ng, 100 ng sont circularisés par ligation avec l'ADN ligase T4 (Life Technologies) dans un volume de 20 µL dans des conditions spécifiées par le fournisseur. Un microlitre de la préparation résultante est amplifié par PCR effectuée sur 30 cycles (1 min. 94°C, 1 min. 60°C, 3 min, 72°C) avec une ADN polymérase Taq (Perkin Elmer) dans des conditions de réaction standards recommandées par le fournisseur. Les amorces utilisées
(MA226 5'-ATGCGATCGGACTGCTGGACA-3' et
MA227 5'-CGCGGGCAATCCACACCTAC-3')
sont déduites de la séquence d'un fragment génomique obtenu précédemment. Un site de restriction Xhol situé entre les 2 sites d'amorçage dans la séquence génomique est utilisé facultativement pour relinéariser l'ADN circulaire avant la PCR inverse, afin de supprimer le stress de torsion et ainsi améliorer l'efficacité de l'amplification initiale du support génomique. Les produits PCR sont séparés par électrophorèse sur gel agarose et les amplicons spécifiques de l'EH *d'Aspergillus* sont identifiés par immunotransfert selon la technique de Southern en utilisant le fragment génomique mentionné ci-dessus en tant que sonde. Les fragments de gène de l'EH *Aspergillus* identifiés de cette façon sont purifiés par électrophorèse sur gel agarose en utilisant le kit Quiaex (Qiagen), et clonés dans le vecteur pGEM-T (Promega) pour les analyses de séquence par la méthode de terminaison de chaîne.

A partir de l'information obtenue de la séquence, 2 amorces
(MA290 5'-cggaattccATGgTCACTGGAGGAGCAATAATTAG-3' et
MA291 5'-ttgaatTCCCTACTTCTGCCACAC-3'; les résidus en lettres capitales sont complémentaires de la séquence support) entourant la région codant la protéine du gène EH sont déduits et utilisés pour amplifier les fragments respectifs de l'ADN génomique et transcrire en inverse l'ARNm avec l'ADN polymérase Pfu de haute fidélité ("Stratagene") sur 40 cycles (1 min. 94°C, 1 min. 50°C, 6 min. 72°C). Les fragments ADN résultant sont digérés avec EcoRI et insérés dans pUC19 (New England Biolabs) pour l'analyse séquentielle finale.

### 3) Expression, purification et analyse de l'époxyde hydrolase recombinante

Pour l'expression recombinante dans *E. coli,* le fragment ADNc de l'époxyde hydrolase *d'Aspergillus* est amplifié avec une ADN polymérase Pfu en utilisant l'amorce MA291 (voir ci-dessus) et l'amorce MA318
(5' gctgaattcacATGTCCGCTCCGTTCGCCAAG-3')
afin d'introduire un site de reconnaissance AfIIII Ncol-compatible (souligné dans le primer MA318) dans le codon d'initiation probable du gène de l'époxyde hydrolase *d'Aspergillus* qui a été révélé par l'analyse des séquences.

Le vecteur d'expression pGEF+ de bactérie, est modifié en introduisant un site multiple de coupure
(5'-CCATGGGAATTCTCGAGATCTAAGCTTATGCATCAGCTGCATGG-3')
dans le site Ncol qui contient le codon de départ du vecteur pGEF+ dans le contexte adapté à un site de liaison du ribosome, en aval du promoteur de l'ARN polymérase T7. Le plasmide résultant est appelé pGEF II dans ce qui suit. Le fragment PCR AfIII/Eco RI de l'EH *d'Aspergillus* est ligaturé dans le site Ncol/Eco RI de pGEF II pour produire la construction d'expression pGEF Asp EH". La souche *E. coli* BL21 (DE 3) (Novagen) est transformée avec pGEF Asp EH et mise dans le milieu LB à 30°C. En phase exponentielle tardive, l'induction de l'expression de la protéine recombinante est réalisée par addition d'isopropyl-β-thiogalactoside (100 µM). Après deux heures, les bactéries sont recueillies par centrifugation, resuspendues dans 0,02 volumes de culture du tampon STE (Tris-HCl, 10 mM, chlorure de sodium, 100 mM, acide éthylènediamine tétraacétique, 1 mM, pH 7,4) et stockées à -70°C. L'activité enzymatique est déterminée par transformation de l'énantiomère R de l'oxyde de *para*-nitrostyrène en diol correspondant. La réaction est conduite à une concentration de substrat de 880 µM dans 500 µL STE à 37°C pendant 30 min., en présence de 10 µL d'acétonitrile qui est utilisé comme solvant de l'oxyde de *para*-nitrostyrène.

La réaction de conversion est terminée par extraction du substrat avec un volume égal de chloroforme. Dans ces conditions, plus de 99,9 % du substrat est extrait dans la phase organique et 60 % du diol est retrouvé dans la phase aqueuse.

Le substrat de conversion est quantifié par addition de 400 µL de surnageant à 800 µL d'eau et en lisant la densité optique à 277 nM, avec le coefficient d'extinction molaire du produit étant 9,1 x 10³ M⁻¹ cm⁻¹. L'époxyde hydrolase *d'Aspergillus* est purifiée jusqu'à homogénéité par une procédure en 3 étapes, selon la méthode décrite ci-dessus.

Les anticorps dirigés contre la protéine purifiée ont été obtenus en immunisant des lapins selon la technique décrite par Friedberg et al., 1991. La protéine purifiée est analysée par électrophorèse sur gel polyacrylamide SDS suivi par un marquage au bleu Coomassie ou par immunotransfert selon les procédures précédemment publiées.

### 4) Construction et analyse des mutants d'époxyde hydrolase

Une mutagenèse dirigée contrôlée par PCR de l'ADNc d'époxyde hydrolase *d'Aspergillus* est conduite par la méthode de Tomic et al., 1990, tel que décrit précédemment pour les époxydes hydrolases solubles de mammifères et les époxydes hydrolases microsomales (Arand et al., 1996; Arand et al., 1999).

Des amorces ont été utilisées pour l'introduction des différentes mutations. Les mutations affectant le nucléophile catalytique Asp¹⁹² sont introduites en échangeant la cassette interne Ncol de l'ADNc de l'époxyde hydrolase *d'Aspergillus* contre le fragment PCR-modifié.

De même, des mutations ciblant les résidus du système de relais de charge, à savoir Asp³⁴⁸ et His³⁷⁴, sont introduites en substituant un fragment XhoI avec le fragment respectif PCR. Les modifications PCR sont générées en utilisant la polymérase ADN Pfu afin de minimiser l'introduction de modifications de séquence non voulues. Tous les fragments PCR-générés sont finalement séquencés pour s'assurer de leur exactitude. Après expression recombinante, la solubilité des protéines mutantes est testée, ce qui représente un indicateur de leur intégrité structurelle. Après sonication des culots bactériens, la suspension résultante est centrifugée à 10,000 g, le culot et le surnageant sont contrôlés pour la présence de l'époxyde hydrolase *d'Aspergillus* par immunotransfert. L'activité enzymatique est contrôlée dans le surnageant tel que décrit précédemment.

### II) Résultats

L'isolement du gène de l'époxyde hydrolase (EH) *d'Aspergillus* et l'ADNc via une PCR inverse, ont été obtenus. Il a été difficile d'obtenir des fragments amplifiés spécifiques en utilisant des enzymes de restriction avec des sites de reconnaissance hexamérique pour la digestion de l'ADN génomique.

Ceci semble dû à deux erreurs d'appariement dans l'amorce MA226, par comparaison à la séquence d'origine naturelle, qui affaiblit l'amplification des produits longs, mais ne pose pas de problème quand on utilise les enzymes de restriction avec des séquences de reconnaissance tétramériques. Cependant, le premier fragment obtenu après une restriction par BamHI de l'ADN semble être artificiellement tronqué, ce qui est une conséquence de l'amorçage interne de l'amplicon initial. Par conséquent, la région 3' de 1'EH *d'Aspergillus* en aval de la séquence génomique est manquante dans ce fragment et doit être obtenue séparément dans une seconde expérience PCR inverse.

L'époxyde hydrolase *d'Aspergillus* est clairement reliée aux mEHs des mammifères, bien que cette enzyme soit unique sous plusieurs aspects.

Premièrement, c'est une enzyme soluble n'ayant pas de séquence d'ancrage dans la membrane, contrairement aux époxydes hydrolases microsomales de mammifères (mEHs), et leurs correspondants chez les arthropodes.

Deuxièmement, l'EH *d'Aspergillus* a un pouvoir de conversion beaucoup plus élevé avec l'oxyde de *para*-nitrostyrène, que ceux des époxydes hydrolases de mammifères avec leurs substrats.

Alors que l'époxyde hydrolase microsomale (mEH) de rat a une activité spécifique avec ses substrats modèles oxyde de styrène et oxyde de benzo[α]pyrène d'environ 500 nmoles converties par minute et milligramme d'enzyme pure, l'époxyde hydrolase d'*Aspergillus* hydrolyse 100 µmol d'oxyde de styrène 4-nitro par minute et milligramme d'enzyme. Le nombre de conversion de mEH de rat a été augmenté par un facteur de 30 en substituant le résidu acide du système de relais de charge de son site catalytique, à savoir Glu⁴⁰⁴, par l'acide aspartique. De façon intéressante, le résidu correspondant dans l'époxyde hydrolase native *Aspergillus* est déjà un acide aspartique, par opposition au fait que l'acide glutamique occupe cette position dans toutes les autres enzymes mEH. La substitution de Asp³⁴⁸ catalytique dans l'époxyde hydrolase *d'Aspergillus* par Glu conduit à une chute modérée du Vmax d'un facteur de seulement 2. Dans le même temps, le K_{M} a chuté d'un facteur de 3. Une possible explication de cette observation pourrait être celle d'une inversion dans l'étape limitant le taux de conversion de la réaction enzymatique. Dans les mEH et sEH de mammifères la seconde étape hydrolytique de la réaction enzymatique semble être une étape limitant le taux de conversion. Dans de telles conditions, c'est à dire lorsque la constante de vitesse de la formation de l'ester intermédiaire k₁ est beaucoup plus important que la constante k₂ pour l'etape hydrolytique, la réduction de Vmax due à un k₂ réduit se fait en parallèle d'une réduction similaire de K_{M}, parce que K_{M} = K_{D}k₂/(k₁+k₂). Cependant, si, initialement k₁ limite le taux, et k₂ est beaucoup plus important, l'expression k₂/(k₁+k₂) sera approximativement égale à 1 et K_{M} est égal à K_{D}. Une réduction de Vmax de moitié due à une modulation du système de relais de charge, c'est-à-dire de la partie importante du site catalytique pour la deuxième étape de la réaction enzymatique, est probablement due à une forte diminution de k₂ allant jusqu'à la moitié de la valeur de k₁. En conséquence, k₂/(k₁+k₂) serait maintenant proche de 1/3, c'est-à-dire exactement la valeur observée pour le mutant Asp³⁴⁸Glu de l'époxyde hydrolase (EH) *d'Aspergillus.* Ainsi, ces résultats sont compatibles avec le fait que dans le cas de EH *d'Aspergillus* avec l'oxyde de *para*-nitrostyrène comme substrat, k₂ est plus grand que k₁, une situation qui n'existe plus lors de la substitution de Asp³⁴⁸ par Glu. Ceci correspondrait exactement au scénario observé avec les mEH de mammifères.

La structure du gène de l'époxyde hydrolase *d'Aspergillus* est très complexe, si on se base sur la simplicité de l'organisme d'origine. Alors que la taille moyenne des introns identifiés est d'environ 60 pb, et donc est en accord avec celle de beaucoup d'autres gènes *d'Aspergillus,* par contre, le nombre d'introns dans le gène *Aspergillus,* 8 en tout, est anormalement élevé.

Aucun des exons/introns n'est conservé entre champignons et mammifères, en dépit du nombre identique d'introns dans les 2 organismes. Les gènes de champignon et de mammifère ont tous les deux un premier exon non-codant. Chez le rat, l'existence d'au moins 3 alternatives pour le premier exon a été notée. Ici, le premier exon non-codant permet l'usage alternatif de différents promoteurs pour la synthèse de protéines identiques.

### C) exemples d'application

### Exemple 1

15g d'Oxyde de 1,1-diéthoxybut-3-ène (94 mmoles soit une concentration de 0,3 mole par litre de milieu réactionnel) sont ajoutés à 300 ml de tampon phosphate (pH 8, 0.1M). La température est amenée à 4°C et 1.2g d'enzyme purifiée (native) sont additionnés. Après 30 heures d'agitation à 4°C l'époxyde résiduel est extrait avec du pentane. L'évaporation du solvant suivie d'une distillation permet d'isoler 4.5g de (*S*)-époxyde (Rdt = 30%, ee = 98%). Une extraction en continu de la phase aqueuse avec du dichlorométhane permet d'isoler après purification sur une colonne de silice 9g de (R)-diol (Rdt = 54%, ee = 47%).

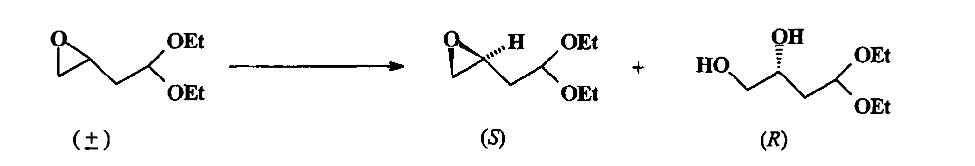

### Exemple 2

6g de *para*-Bromo-α-méthylstyrène oxyde (28 mmoles soit une concentration de 0,35 mole par litre de milieu réactionnel) sont ajoutés à 75 ml de tampon phosphate (pH 8, 0.1M). La température est amenée à 4°C et 0.35g d'enzyme purifiée (native) sont additionnés. Après 8 jours d'agitation à 0°C l'époxyde résiduel est extrait avec du pentane. L'évaporation du solvant suivie d'une distillation permet d'isoler 2.3g de (*S*)-époxyde (Rdt = 39%, ee = 99.7%). Une extraction en continu de la phase aqueuse avec du dichlorométhane permet d'isoler après purification sur une colonne de silice 3.19g de (R)-diol (Rdt = 49%, ee = 96%).

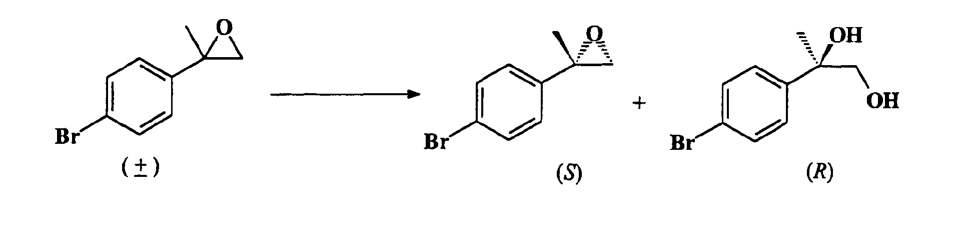

### Exemple 3

4g de *para*-Chlorostyrène oxyde (26 mmoles soit une concentration de 2 moles par litre de milieu réactionnel) sont ajoutés à 9 ml de tampon phosphate (pH 7, 0.1M). La température est amenée à 0°C et 2.3g d'enzyme purifiée (native) sont additionnés. Après 8 heures d'agitation à 0°C l'époxyde résiduel est extrait avec du pentane. L'évaporation du solvant suivie d'une distillation permet d'isoler 1.9g de (*S*)-époxyde (Rdt = 47% , ee = 99%). Une extraction de la phase aqueuse avec de l'acétate d'éthyle permet d'isoler après purification sur une colonne de silice 2.15g de (R)-diol (Rdt = 48%, ee = 92%).

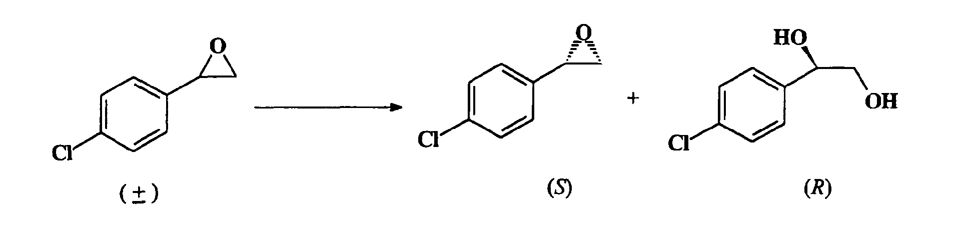

### Exemple 4

4g de *para*-Nitrostyrène oxyde (24 mmoles soit une concentration de 0,3 mole par litre de milieu réactionnel) dissous dans 15 ml de DMSO sont ajoutés à 60 ml de tampon phosphate (pH 7, 0.1M). La température est amenée à 27°C et 0.7g d'enzyme purifiée (native) sont additionnés. Après 32 heures d'agitation le milieu réactionnel est saturé avec du NaCl puis extrait en continu avec du dichlorométhane. L'évaporation du solvant suivie d'une chromatographie sur silice permet d'isoler 1.8g de (*S*)-époxyde (Rdt = 45%, ee = 96%) et 2.3g de (R)-diol (Rdt = 52 % , ee = 86%).

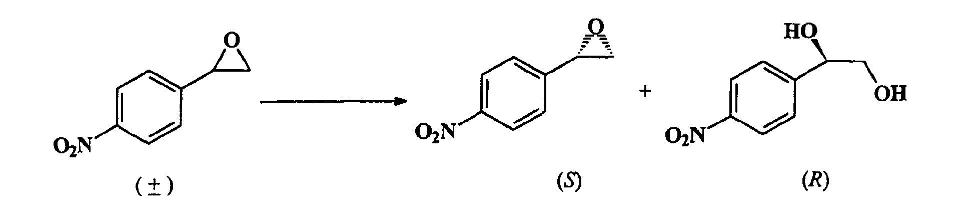

### Exemple 5

1.5g de *para*-isobutyl-α-méthylstyrène oxyde (7,9 mmoles soit une concentration de 0,25 mole par litre de milieu réactionnel) sont ajoutés à 30 ml de tampon tris (pH 8, 0.4M). La température est amenée à 4°C et 2.6g d'enzyme purifiée (native) sont additionnés. Après 24 jours d'agitation à 4°C l'époxyde résiduel est extrait avec du pentane. L'évaporation du solvant suivie permet d'obtenir le (*S*)-époxyde (ee = 96%) non purifié. Une extraction de la phase aqueuse avec de l'éther permet d'isoler après purification sur une colonne de silice 0.91g de (R)-diol (Rdt = 55%, ee = 70%).

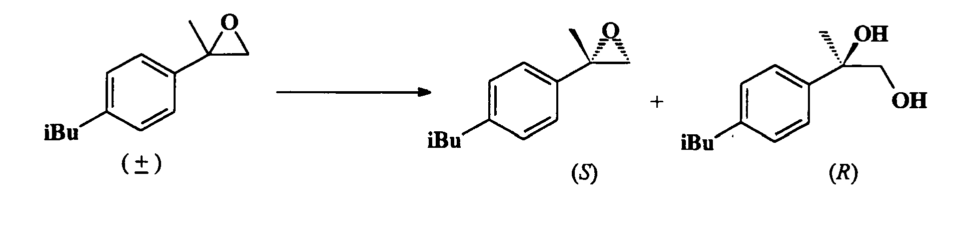

### Exemple 6

1g de phényl glycidyl éther (6,7 mmoles soit une concentration de 3,3 moles par litre de milieu réactionnel) est ajouté à 1 ml de tampon phosphate (pH 7, 0.1M). La température est amenée à 27°C et 25 mg d'enzyme recombinante purifiée sont additionnés. Après 15 heures d'agitation à 27°C la totalité de l'époxyde est transformé en diol correspondant racémique. Une extraction avec de l'acétate d'éthyle permet d'isoler ce diol avec un rendement quantitatif.

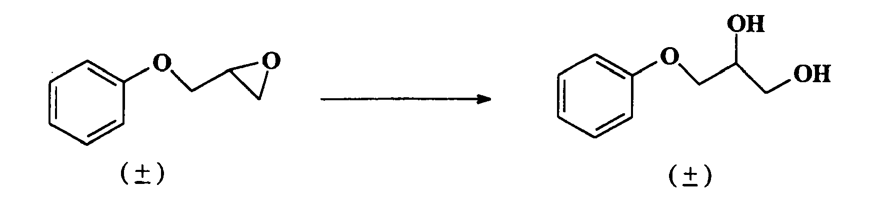

### BIBLIOGRAPHIE

- Audier H.E., Dupin J.F., Jullien J. (1968) Bull. Chem. Soc., 9, 3844-3847.
- Arand M., Wagner H., Oesch F. (1996) J. Biol. Chem., 271, 4223-4229
- Arand M., Müller F., Mecky A., Hinz W., Urban P., Pompon D., Kellner R., Oesch F. (1999) Biochem. J. , 337, 37-43
- Archelas A., Furstoss R. (1997) Ann. Rev. Microbiol., 51, 491-525
- Archelas A., Furstoss R. (1998) Trends in Biotechnology, 16, 108-116
- Beetham J. K., Grant D., Arand M., Garbarino J., Kiyosue T., Pinot F., Oesch F., Belknap W. R., shinozaki K., Hammock B. D. (1995) DNA Cell Biol., 14, 67-71
- Blée E., Schuber F. (1992) Biochem. J. , 282, 711-714
- Borhan B., Jones A. D., Pinot F., Grant D. F., Kurth M. J., Hammock B. D. (1995) Anal. Biochem., 231, 188-200
- Chomczynski P., Sacchi N. (1986) Anal. Biochem., 162, 156-159
- Dansette P.M., Makedonska V.B., Jerina D.M. (1978) Arch. Biochem. Biophys. 187, 290-298
- Delaage M. (1968) Biochim. Biophys. Acta, 168, 443-445.
- Friedberg T., Kissel W., Arand M., Oesch F. (1991) In Methods in Enzymology (Waterman M. R. and Johnson E. F., eds) Vol. 206, pp. 193-201, Academic Press, New York
- Hammock B. D., Grant D. F., Storms D. H. (1997) In Comprehensive Toxicology (Sipes, I., McQueen C. and Gandolfi, A., Eds), pp 283-305, Pergamon Press, Oxford
- Laemmli U. K. (1970) Nature, 227, 680-685
- Lowry O. H., Rosebrough N.J., Farr A. L., Randall R. J.(1951) J. Biol. Chem., 193, 265-275
- Misawa E., Chan Kwo Chion C. K. C., Archer I. W., Woodland M. P., Zhou N. Y., Carter S., Widdowson D. A., Leak D. A. (1998) Eur. J. Biochem., 253, 173-183
- Nellaiah H., Morisseau C., Archelas A., Furstoss R., Baratti J. C. (1996) Biotech. Bioeng., 49, 70-77
- Pedragosa-Moreau S., Archelas A., Furstoss R. (1993) J. Org. Chem. 58, 5533-5536
- Pedragosa-Moreau S., Archelas A., Furstoss R. (1995) Bull. Soc. Chim. Fr. 132, 769-800
- Pedragosa-Moreau S., Archelas A., Furstoss R. (1996) J. Org. Chem, 61, 7402-7407
- Pedragosa-Moreau S., Morisseau C., Zylber J., Baratti J.C., Archelas A., Furstoss R. Tetrahedron (1997) 53, 9707-9714
- Rink R., Fennema M., Smids M., Dehmel U., Janssen D. B. (1997) J. Biol. Chem., 272, 14650-14657
- Schurig V., Betschinger F. (1992) Chem. Rev. 873-888
- Tomic M., Sunjeravic I., Savtchenko E.S., Blumenberg M. (1990) Nucleic Acids Res. , 18, 1656
- Touhara K., Prestwitch G. D. (1993) J. Biol. Chem, 268, 19604-19609
- Westkaemper R.B., Hanzlik R.P. (1980) Anal. Biochem., 102, 63-67
- Westkaemper R.B., Hanzlik R.P. (1981) Arch. Biochem. Biophys. 208, 195-204
- Wixtrom R.N., Hammock B.D. (1985) In Biochemical Pharmacology and Toxicology (Zakim D. and Vessey D.A., Eds) pp. 1-93, John Willey & Sons, New-York.

### LISTE DE SEQUENCES

<110> C.N.R.S.
<120> PROTEINES D'ORIGINE FONGIQUE ET DERIVEES, LEURS PROCEDES D'OBTENTION, ET LEURS UTILISATIONS, NOTAMMENT POUR LA PREPARATION DE MOLECULES ENANTIOMERIQUEMENT PURES
<130> EPOXSL
<140>
   <141>
<160> 2
<170> PatentIn Ver. 2.1
<210> 1
   <211> 1197
   <212> ADN
   <213> Aspergillus niger
<220>
   <221> CDS
   <222> (1)..(1197)

Séquence nucléotidique SEQ ID NO : 1
   <400> 1 
atg tcc gct ccg ttc gcc aag ttt ccc tcg tcg gcg agc att tcg cct    48
Met Ser Ala Pro Phe Ala Lys Phe Pro Ser Ser Ala Ser Ile Ser Pro
1               5                   10                  15

aat cct ttc acg gtc tct atc ccg gat gaa cag ttg gat gac ttg aaa    96
Asn Pro Phe Thr Val Ser Ile Pro Asp Glu Gln Leu Asp Asp Leu Lys
            20                  25                  30

acc ctc gtc cga ctg tcc aag att gct cct ccc acc tat gag agc ctg    144
Thr Leu Val Arg Leu Ser Lys Ile Ala Pro Pro Thr Tyr Glu Ser Leu
        35                  40                  45

caa gcg gat ggc cgg ttt ggc atc act tct gaa tgg ctg aca act atg    192
Gln Ala Asp Gly Arg Phe Gly Ile Thr Ser Glu Trp Leu Thr Thr Met
    50                  55                  60

cgg gag aaa tgg ctc tcg gag ttt gac tgg cga cca ttt gaa gct cga    240
Arg Glu Lys Trp Leu Ser Glu Phe Asp Trp Arg Pro Phe Glu Ala Arg
65                  70                  75                  80

ctg aac tct ttc cct cag ttt act aca gag atc gag ggt ctc acg att    288
Leu Asn Ser Phe Pro Gln Phe Thr Thr Glu Ile Glu Gly Leu Thr Ile
                85                  90                  95

cac ttt gct gct ctc ttc tcc gag agg gag gat gct gtg cct atc gca    336
His Phe Ala Ala Leu Phe Ser Glu Arg Glu Asp Ala Val Pro Ile Ala
            100                 105                 110

ttg ctc cat ggt tgg ccc ggc agc ttc gtt gag ttc tac cca atc ctg    384
Leu Leu His Gly Trp Pro Gly Ser Phe Val Glu Phe Tyr Pro Ile Leu
        115                 120                 125
 
cag cta ttc cgg gag gag tac acc cct gag act ctg cca ttc cat ctg 432
Gln Leu Phe Arg Glu Glu Tyr Thr Pro Glu Thr Leu Pro Phe His Leu
    130                 135                 140

gtt gtt ccg tcc ctt cct ggg tat act ttt tca tct ggt ccc ccg ctg 480
Val Val Pro Ser Leu Pro Gly Tyr Thr Phe Ser Ser Gly Pro Pro Leu
145                 150                 155                 160

gac aag gac ttc ggc ttg atg gac aac gcc cgg gtc gta gac cag ttg 528
Asp Lys Asp Phe Gly Leu Met Asp Asn Ala Arg Val Val Asp Gln Leu
                165                 170                 175

atg aag gac ctc ggg ttc gga agt ggt tat att att cag gga ggt gat 576
Met Lys Asp Leu Gly Phe Gly Ser Gly Tyr Ile Ile Gln Gly Gly Asp
            180                 185                 190

att ggt agc ttt gtt gga cga ctg ttg ggc gtg ggt ttc gac gcc tgc 624
Ile Gly Ser Phe Val Gly Arg Leu Leu Gly Val Gly Phe Asp Ala Cys
        195                 200                 205

aaa gcg gtt cat ttg aac ctg tgc gca atg agg gct ccc cct gag ggc 672
Lys Ala Val His Leu Asn Leu Cys Ala Met Arg Ala Pro Pro Glu Gly
    210                 215                 220

ccg tca att gag agc ttg tcc gca gcg gag aag gag gga atc gcg cga 720
Pro Ser Ile Glu Ser Leu Ser Ala Ala Glu Lys Glu Gly Ile Ala Arg
225                 230                 235                 240

atg gag aag ttc atg acc gat ggc tta gct tat gcc atg gag cac agt 768
Met Glu Lys Phe Met Thr Asp Gly Leu Ala Tyr Ala Met Glu His Ser
                245                 250                 255

act cgg ccc agt act att ggc cac gtg ctg tcc agc agt ccg atc gca 816
Thr Arg Pro Ser Thr Ile Gly His Val Leu Ser Ser Ser Pro Ile Ala
            260                 265                 270

tta ctt gca tgg att ggt gag aaa tat ctc caa tgg gtg gat aaa ccc 864
Leu Leu Ala Trp Ile Gly Glu Lys Tyr Leu Gln Trp Val Asp Lys Pro
        275                 280                 285

ctc cct tct gag acc atc ctc gag atg gtg agc ctg tat tgg ctg acg 912
Leu Pro Ser Glu Thr Ile Leu Glu Met Val Ser Leu Tyr Trp Leu Thr
    290                 295                 300

gaa agt ttc ccg cgg gca att cat acc tac cgc gag act acc cca act 960
Glu Ser Phe Pro Arg Ala Ile His Thr Tyr Arg Glu Thr Thr Pro Thr
305                 310                 315                 320

gcc tcc gct ccc aat gga gcg aca atg ctt cag aag gaa tta tat att 1008
Ala Ser Ala Pro Asn Gly Ala Thr Met Leu Gln Lys Glu Leu Tyr Ile
                325                 330                 335

cac aag ccg ttt ggg ttc tcc ttc ttc ccc aag gac ctt tgt cct gtg 1056
His Lys Pro Phe Gly Phe Ser Phe Phe Pro Lys Asp Leu Cys Pro Val
            340                 345                 350

cct cgg agc tgg att gct aca acg gga aat cta gta ttc ttc cgg gat 1104
Pro Arg Ser Trp Ile Ala Thr Thr Gly Asn Leu Val Phe Phe Arg Asp
        355                 360                 365
 
cat gca gag gga gga cac ttt gcc gca ttg gag cgt cca cgc gag ctg    1152
His Ala Glu Gly Gly His Phe Ala Ala Leu Glu Arg Pro Arg Glu Leu
    370                 375                 380

aag acc gac ctg aca gca ttt gtc gag cag gtg tgg cag aag tag        1197
Lys Thr Asp Leu Thr Ala Phe Val Glu Gln Val Trp Gln Lys
385                 390                 395
Séquence peptidique SEQ ID NO : 2
   <210> 2
      <211> 399
      <212>
      <213> Aspergillus niger
   <400> 2

Met Ser Ala Pro Phe Ala Lys Phe Pro Ser Ser Ala Ser Ile Ser Pro
1               5                   10                  15

Asn Pro Phe Thr Val Ser Ile Pro Asp Glu Gln Leu Asp Asp Leu Lys
            20                  25                  30

Thr Leu Val Arg Leu Ser Lys Ile Ala Pro Pro Thr Tyr Glu Ser Leu
        35                  40                  45

Gln Ala Asp Gly Arg Phe Gly Ile Thr Ser Glu Trp Leu Thr Thr Met
    50                  55                  60

Arg Glu Lys Trp Leu Ser Glu Phe Asp Trp Arg Pro Phe Glu Ala Arg
65                  70                  75                  80

Leu Asn Ser Phe Pro Gln Phe Thr Thr Glu Ile Glu Gly Leu Thr Ile
                85                  90                  95

His Phe Ala Ala Leu Phe Ser Glu Arg Glu Asp Ala Val Pro Ile Ala
            100                 105                 110

Leu Leu His Gly Trp Pro Gly Ser Phe Val Glu Phe Tyr Pro Ile Leu
        115                 120                 125

Gln Leu Phe Arg Glu Glu Tyr Thr Pro Glu Thr Leu Pro Phe His Leu
    130                 135                 140

Val Val Pro Ser Leu Pro Gly Tyr Thr Phe Ser Ser Gly Pro Pro Leu
145                 150                 155                 160

Asp Lys Asp Phe Gly Leu Met Asp Asn Ala Arg Val Val Asp Gln Leu
                165                 170                 175

Met Lys Asp Leu Gly Phe Gly Ser Gly Tyr Ile Ile Gln Gly Gly Asp
            180                 185                 190

Ile Gly Ser Phe Val Gly Arg Leu Leu Gly Val Gly Phe Asp Ala Cys
        195                 200                 205

Lys Ala Val His Leu Asn Leu Cys Ala Met Arg Ala Pro Pro Glu Gly
    210                 215                 220

Pro Ser Ile Glu Ser Leu Ser Ala Ala Glu Lys Glu Gly Ile Ala Arg
225                 230                 235                 240

Met Glu Lys Phe Met Thr Asp Gly Leu Ala Tyr Ala Met Glu His Ser
                245                 250                 255
Thr Arg Pro Ser Thr Ile Gly His Val Leu Ser Ser Ser Pro Ile Ala
            260                 265                 270
Leu Leu Ala Trp Ile Gly Glu Lys Tyr Leu Gln Trp Val Asp Lys Pro
        275                 280                 285
Leu Pro Ser Glu Thr Ile Leu Glu Met Val Ser Leu Tyr Trp Leu Thr
    290                 295                 300
Glu Ser Phe Pro Arg Ala Ile His Thr Tyr Arg Glu Thr Thr Pro Thr
305                 310                 315                 320
Ala Ser Ala Pro Asn Gly Ala Thr Met Leu Gln Lys Glu Leu Tyr Ile
                325                 330                 335
His Lys Pro Phe Gly Phe Ser Phe Phe Pro Lys Asp Leu Cys Pro Val
            340                 345                 350
Pro Arg Ser Trp Ile Ala Thr Thr Gly Asn Leu Val Phe Phe Arg Asp
        355                 360                 365
His Ala Glu Gly Gly His Phe Ala Ala Leu Glu Arg Pro Arg Glu Leu
    370                 375                 380
Lys Thr Asp Leu Thr Ala Phe Val Glu Gln Val Trp Gln Lys
385                 390                 395

## Revendications

1. Protéine ayant une activité époxyde hydrolase, **caractérisée en ce qu'**elle comprend un fragment d'au moins 10 acides aminés contigus dans la région délimitée par les acides aminés situés aux positions 1 et 339 de la séquence SEQ ID NO : 2, laquelle est obtenue par purification à homogénéité électrophorétique à partir de cellules de champignons, ou par mise en culture de cellules hôtes transformées par une séquence nucléotidique codant pour la protéine fongique susmentionnée.

2. Protéine selon la revendication 1, **caractérisée en ce qu'**elle comprend la séquence SEQ ID NO : 2.

3. Protéine selon la revendication 1 ou 2, **caractérisée en ce qu'**elle correspond à une époxyde hydrolase fongique purifiée à homogénéité électrophorétique, telle qu'obtenue par extraction et purification à partir de cultures de cellules de champignons de l'espèce *Aspergillus.*

4. Protéine selon l'une des revendications 1 à 3, **caractérisée en ce qu'**elle correspond à l'époxyde hydrolase fongique purifiée à homogénéité électrophorétique représentée par SEQ ID NO : 2, telle qu'obtenue par extraction et purification à partir de cultures de cellules de souches *d'Aspergillus niger* ou d'*Aspergillus turingensis.*

5. Protéine selon la revendication 1 ou 2, **caractérisée en ce qu'**elle correspond à une époxyde hydrolase fongique recombinante, telle qu'obtenue par purification à homogénéité électrophorétique par transformation de cellules hôtes appropriées à l'aide de vecteurs contenant :
- la séquence nucléotidique SEQ ID NO : 1 codant l'époxyde hydrolase représentée par SEQ ID NO : 2, ou toute séquence dérivée de SEQ ID NO : 1 par dégénérescence du code génétique, et codant l'époxyde hydrolase représentée par SEQ ID NO:2,
- ou tout fragment de la séquence SEQ ID NO : 1, codant une enzyme possédant une activité époxyde hydrolase, ledit fragment étant constitué d'au moins environ 20 nucléotides contigus dans la région délimitée par les nucléotides situés aux positions 1 et 1197 de la séquence SEQ ID NO : 1.

6. Protéine selon la revendication 5, **caractérisée en ce qu'**elle correspond à l'époxyde hydrolase fongique recombinante représentée par SEQ ID NO : 2, telle qu'obtenue par transformation de cellules hôtes appropriées à l'aide de vecteurs contenant la séquence nucléotidique SEQ ID NO : 1, ou toute séquence dérivée de SEQ ID NO : 1 par dégénérescence du code génétique, et codant l'époxyde hydrolase représentée par SEQ ID NO : 2.

7. Séquence nucléotidique codant une protéine à activité époxyde hydrolase telle que définie l'une des revendications 1 à 6.

8. Séquence nucléotidique selon la revendication 7, **caractérisée en ce qu'**elle comprend :
- la séquence représentée par SEQ ID NO : 1 codant l'époxyde hydrolase représentée par SEQ ID NO : 2,
- ou toute séquence dérivée de la séquence SEQ ID NO : 1 par dégénérescence du code génétique, et codant l'époxyde hydrolase représentée par SEQ ID NO : 2,
- ou tout fragment de la séquence SEQ ID NO : 1, codant une enzyme possédant une activité époxyde hydrolase, ledit fragment étant constitué d'au moins environ 20 nucléotides contigus dans la région délimitée par les nucléotides situés aux positions 1 et 1197 de la séquence SEQ ID NO : 1,
- ou toute séquence nucléotidique complémentaire des séquences ou fragments susmentionnés,
les séquences ou fragments susmentionnés étant sous forme simple brin ou double brin.

9. Vecteur, notamment plasmide, contenant une séquence nucléotidique selon la revendication 7 ou 8.

10. Cellule hôte transformée par un vecteur selon la revendication 9.

11. Cellule hôte selon la revendication 10 choisie parmi les bactéries, les virus, les levures, les champignons, les plantes ou les cellules de mammifères, ladite cellule hôte étant transformée, à l'aide d'un vecteur selon la revendication 9.

12. Utilisation de protéines à activité époxyde hydrolase définies dans l'une des revendications 1 à 6, en tant que biocatalyseurs enzymatiques dans le cadre de la mise en oeuvre de procédés de préparation d'époxydes ou de diols vicinaux énantiomériquement purs.

13. Utilisation des biocatalyseurs selon la revendication 12, pour réaliser l'hydrolyse d'époxydes achiraux ou chiraux et l'obtention de diols achiraux, racémiques ou optiquement enrichis, à savoir sans mettre en oeuvre de réactif acide ou basique minéral ou organique, notamment en milieu aqueux tamponné ou non tamponné et/ou en présence de solvants organiques miscibles ou non miscibles à l'eau.

14. Procédé de préparation d'époxydes et/ou de diols énantiomériquement purs respectivement de formules (II) et (III) suivantes 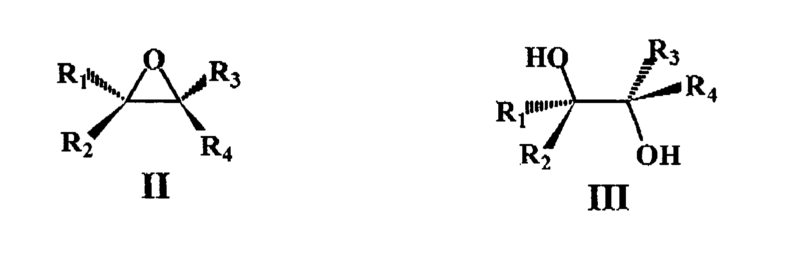 dans lesquelles R₁, R₂, R₃ et R₄ représentent des groupes quelconques
ledit procédé comprenant une étape de traitement d'un mélange d'époxydes diastéréoisomères, ou d'un époxyde chiral sous forme racémique, ou d'un époxyde prochiral de formule (I) suivante : 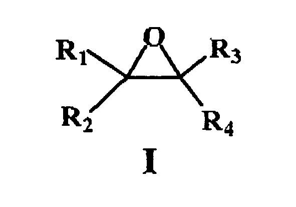 avec une protéine à activité époxyde hydrolase selon l'une des revendications 1 à 6, ou avec des cellules hôtes selon la revendication 10 ou 11 exprimant une protéine à activité époxyde hydrolase selon l'une des revendications 1 à 6, ce qui conduit à l'obtention :
- d'un mélange des composés de formule (II) et (III) susmentionnés, lesdits composés de formule (II) et (III) pouvant, le cas échéant, être séparés par une étape supplémentaire de purification,
- ou du seul composé de formule (III) susmentionnée.

15. Procédé de préparation d'une protéine à activité époxyde hydrolase recombinante selon la revendication 5 ou 6, **caractérisé en ce qu'**il comprend une étape de transformation de cellules hôtes, choisies parmi les bactéries, les virus, les levures, les champignons, les plantes ou les cellules de mammifères, avec un vecteur selon la revendication 9, et une étape de purification de l'époxyde hydrolase recombinante produite par lesdites cellules.

16. Procédé de préparation d'une protéine à activité époxyde hydrolase purifiée à homogénéité électrophorétique selon la revendication 3 ou 4, ledit procédé comprenant :
- une étape d'extraction de l'enzyme à partir de cultures cellulaires de champignons, tels que les champignons de l'espèce *Aspergillus,* suivie d'une étape de centrifugation à faible vitesse, récupération du surnageant, et, le cas échéant concentration,
- une étape de purification de l'enzyme à partir de l'extrait obtenu à l'étape précédente, par passages successifs sur des colonnes de DEAE-Sepharose, Phényl-Sepharose, Mono Q et Superose 12.

## Claims

1. Protein endowed with an epoxide hydrolase activity, **characterized in that** it includes a fragment of at least 10 contiguous amino acids located in the area delimited by the amino acids of positions 1 and 339 of SEQ ID NO: 2, the aforementioned protein being obtained by purification to electrophoretic homogeneity from cells of fungi, or by growing host cells transformed by a nucleotide sequence encoding for the aforementioned protein of fungi.

2. Protein according to claim 1, **characterized in that** it includes SEQ ID NO: 2.

3. Protein according to claim 1 or 2, **characterized in that** it is a fungal epoxide hydrolase purified to electrophoretic homogeneity, as obtained by extraction and purification from cultures of fungal cells of the species *Aspergillus.*

4. Protein according to claim 1 to 3, **characterized in that** it is a fungal epoxide hydrolase purified to electrophoretic homogeneity, represented by SEQ IDN °: 2, as obtained by extraction and purification from cultures of fungal cells of the species *Aspergillus niger* or *Aspergillus ruringensis.*

5. Protein according to claim 1 or 2, **characterized in that** it is a recombinant epoxide hydrolase of fungi, as obtained by purification to electrophoretic homogeneity, by transforming appropriate host cells by means of vectors containing:
- the nucleotide sequence SEQ ID NO: 1 encoding the epoxide hydrolase represented by SEQ ID NO: 2, or any derivative sequence of SEQ ID NO: 1 by degeneration of the genetic code, and encoding the epoxide hydrolase represented by SEQ ID NO: 2,
- or any fragments of SEQ ID NO: 1, encoding the enzyme endowed with an epoxide hydrolase activity, said fragment consisting of at least about 20 contiguous nucleotides located in positions 1 and 1197 of the sequence SEQ ID NO: 1.

6. Protein according to claim 5, **characterized in that** it is a recombinant epoxide hydrolase of fungi represented by SEQ ID NO: 2, as obtained by transforming appropriate host cells by means of vectors containing the nucleotide sequence SEQ ID NO: 1, or any derivative sequence of SEQ ID NO: 1 by degeneration of the genetic code, and encoding the epoxide hydrolase represented by SEQ ID NO: 2.

7. Nucleotide sequence encoding a protein endowed with an epoxide hydrolase activity as defined by claims 1 to 6.

8. Nucleotide sequence of claim 7, **characterized in that** it includes:
- the sequence represented by SEQ ID NO: 1 encoding the epoxide hydrolase represented by SEQ ID NO: 2,
- or any sequence derived from SEQ ID NO: 1 by degeneration of the genetic code, and encoding the epoxide hydrolase represented by SEQ ID NO: 2.
- or any fragments of SEQ ID NO: 1, encoding an enzyme with an epoxide hydrolase activity, the fragment consisting of at least about 20 contiguous nucleotides located in positions 1 and 1197 of the sequence SEQ ID NO: 1,
- or any complementary sequences to the aforementioned nucleotide sequences or to the aforementioned fragments.,
said nucleotide sequences or said fragments being a single stranded sequence or a double strand sequence.

9. Vector, including plasmid, containing a nucleotide sequence of claim 7 or 8.

10. Host cell transformed by a vector of claim 9.

11. Host cell of claim 10 selected from bacteria, viruses, yeasts, fungi, plants or mammalian cells said host cell being transformed with a vector of claim 9.

12. Use of a protein endowed with an epoxide hydrolase activity as defined in one of claims 1 to 6, as enzymatic biocatalysts in the implementation of methods of preparation of enantiomerically pure epoxides or vicinal diols.

13. Use of biocatalysts according to claim 12, to achieve the hydrolysis of achiral or chiral epoxy and to obtain diols achiraux, racemic or optically enriched, namely without implementing reagent acid or alkaline mineral or organic, particularly in middle buffered aqueous or non-stamped and / or in the presence of organic solvents or mixed immiscible with water.

14. A method of preparation of enantiomerically pure epoxides and/or of diols respectively of the following formulae (II) and (III): 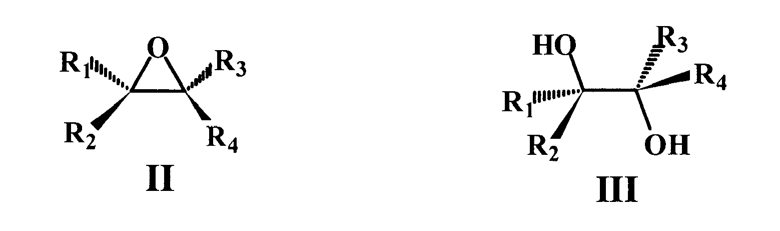 in which R₁, R₂, R₃ and R₄ represent any groups,
said method comprising a stage of treatment of a mixture of diastereoisomeric epoxides, or of a chiral epoxide in racemic form, or of a prochiral epoxide of the following formula (I): 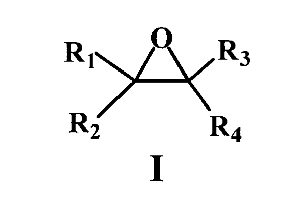 with a protein endowed with epoxide hydrolase activity according to one of the Claims 1 to 6, or with host cells according to claim 10 or 11 expressing a protein endowed with epoxide hydrolase activity according to claim 1 to 6, leading to the obtention of :
- a mixture of aforementioned compounds of formula (II) and (III), said compounds of formula (II) and (III), if necessary, being further separated by a added purification step,
- or just the aforementioned compound of formula (III).

15. A method of preparation of a recombinant protein endowed with an epoxide hydrolase activity according to Claim 5 or 6, **characterized in that** it comprises a stage of transformation of host cells, chosen from the bacteria, viruses, yeasts, fungi, plants or mammalian cells, with a vector according to Claim 9, and a stage of purification of the recombinant epoxide hydrolase produced by the said cells.

16. A method of preparation of a protein with epoxide hydrolase activity according to Claim 3 or 4, the said method comprising:
- a stage of extraction of the enzyme from cellular cultures of fungi, such as fungi of the *Aspergillus* species, by crushing the fungus using a press, followed by a stage of low-speed centrifugation, recovery of the supernatant, and, if required, concentration,
- a stage of purification of the enzyme from the extract obtained in the preceding stage, by successive passages through columns of DEAE-Sepharose, Phenyl-Sepharose, Mono Q and Superose 12.

## Patentansprüche

1. Protein, das eine Epoxidhydrolaseaktivität aufweist, **dadurch gekennzeichnet, dass** es ein Fragment von mindestens 10 aufeinander folgenden Aminosäuren in der Region umfasst, die von den Aminosäuren an den Stellen 1 und 339 der Sequenz SEQ ID NO: 2 begrenzt wird, wobei es durch elektrophoretische Aufreinigung zur Homogenität ausgehend von Pilzzellen erhalten wird oder durch Kultivierung von Wirtszellen, die mit einer Nükleotidsequenz transformiert sind, welche für das obige Protein aus Pilzen kodiert.

2. Protein nach Anspruch 1, **dadurch gekennzeichnet, dass** es die Sequenz SEQ ID NO: 2 umfasst.

3. Protein nach Anspruch 1 oder 2, **dadurch gekennzeichnet, dass** es einer zur Homogenität elektrophoretisch aufgereinigten Epoxidhydrolase aus Pilzen entspricht, wie sie durch Extraktion und Aufreinigung ausgehend von Pilzzellkulturen der Gattung *Aspergillus* erhalten wird.

4. Protein nach einem der Ansprüche 1 bis 3, **dadurch gekennzeichnet, dass** es einer zur Homogenität elektrophoretisch aufgereinigten Epoxidhydrolase nach SEQ ID NO: 2 entspricht, wie sie durch Extraktion und Aufreinigung ausgehend von Pilzzellkulturen der Stämme *Aspergillus niger* oder *Aspergillus turingensis* erhalten wird.

5. Protein nach einem der Ansprüche 1 oder 2, **dadurch gekennzeichnet, dass** es einer rekombinanten Epoxidhydrolase aus Pilzen entspricht, wie sie durch elektrophoretische Aufreinigung zur Homogenität durch Transformation geeigneter Wirtszellen mit Hilfe eines Vektors erhalten wird, der enthält:
- die Nukleotidsequenz SEQ ID NO: 1, die für die durch SEQ ID NO: 2 dargestellte Epoxidhydrolase kodiert oder eine beliebige Sequenz, die durch Degeneration des genetischen Codes von SEQ ID NO: 1 abgeleitet ist und für eine durch SEQ ID NO: 2 dargestellte Epoxidhydrolase kodiert,
- oder ein beliebiges Fragment der Sequenz SEQ ID NO: 1, das für ein Enzym mit einer Epoxidhydrolaseaktivität kodiert, wobei das Fragment von mindestens ungefähr 20 aufeinander folgenden Nukleotiden aus der Region gebildet wird, die durch die Nukleotide an den Positionen 1 und 1197 der Sequenz SEQ ID NO: 1 begrenzt wird.

6. Protein nach Anspruch 5, **dadurch gekennzeichnet, dass** es einer rekombinanten Epoxidhydrolase aus Pilzen entspricht, die durch SEQ ID NO: 2 dargestellt wird, welche durch Transformation von geeigneten Wirtszellen mit Hilfe von Vektoren erhalten wird, welche die Nukleotidsequenz SEQ ID NO: 1 enthalten oder eine beliebige Sequenz, die durch Degeneration des genetischen Codes von SEQ ID NO: 1 abgeleitet ist und für eine durch SEQ ID NO: 2 dargestellte Epoxidhydrolase kodiert.

7. Nukleotidsequenz, die für ein Protein mit Epoxidhydrolaseaktivität, wie in einem der Ansprüche 1 bis 6 definiert, kodiert.

8. Nukelotidsequenz nach Anspruch 7, **dadurch gekennzeichnet, dass** sie umfasst:
- die durch SEQ ID NO: 1 dargestellte Sequenz, die für die durch SEQ ID NO: 2 dargestellte Epoxidhydrolase kodiert,
- oder eine beliebige Sequenz, die durch Degeneration des genetischen Codes von SEQ ID NO: 1 abgeleitet ist und für eine durch SEQ ID NO: 2 dargestellte Epoxidhydrolase kodiert,
- oder ein beliebiges Fragment der Sequenz SEQ ID NO: 1, das für ein Enzym mit einer Epoxidhydrolaseaktivität kodiert, wobei das Fragment von mindestens ungefähr 20 aufeinander folgenden Nukleotiden aus der Region gebildet wird, die durch die Nukleotide an den Positionen 1 und 1197 der Sequenz SEQ ID NO: 1 begrenzt wird,
- oder einer beliebigen Sequenz, die den obigen Sequenzen oder Fragmenten komplementär ist,
wobei die obigen Sequenzen oder Fragmente in einzel- oder doppelsträngiger Form vorliegen.

9. Vektor, insbesondere Plasmid, das eine Nukleotidsequenz nach Anspruch 7 oder 8 enthält.

10. Wirtszelle, die mit einem Vektor des Anspruchs 9 transformiert ist.

11. Wirtszelle nach Anspruch 10, ausgewählt aus Bakterien, Viren, Hefen, Pilzen, Pflanzen oder Säugerzellen, wobei die Wirtszelle mit Hilfe eines Vektors nach Anspruch 9 transformiert ist.

12. Verwendung von Proteinen mit Epoxidhydrolaseaktivität, wie in einem der Ansprüche 1 bis 6 definiert, in der Eigenschaft als enzymatische Biokatalysatoren im Zusammenhang mit dem Einsatz bei der Herstellung von Epoxiden oder enantiomerisch reinen vicinalen Diolen.

13. Verwendung von Biokatalysatoren nach Anspruch 12 zur Hydrolyse von achiralen oder chiralen Epoxiden und zum Erhalt von achiralen, racemischen oder optisch angereicherten Diolen, ohne Einsatz eines sauren oder basischen, mineralischen oder organischen Reagens, insbesondere in wässrigem, gepufferten oder ungepufferten Medium und/oder in Gegenwart von organischen Lösemitteln, die mit Wasser mischbar oder nicht mischbar sind.

14. Verfahren zur Herstellung von Epoxiden und/oder enantiomerisch reinen Diolen nach den folgenden Formeln (II) beziehungsweise (III) 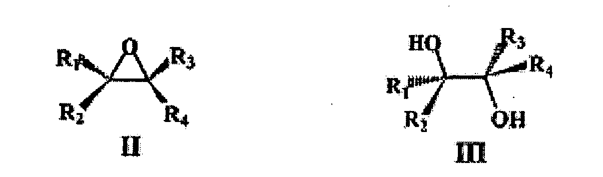 wobei R₁, R₂, R₃ und R₄ beliebige Gruppen darstellen,
wobei das Verfahren einen Schritt der Behandlung eines diastereoisomeren Epoxidgemischs oder eines chiralen Epoxids in racemischer Form oder eines prochiralen Epoxids der folgenden Formel (I) umfasst: 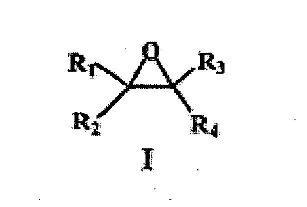 mit einem Protein mit Epoxidhydrolaseaktivität nach einem der Ansprüche 1 bis 6 oder mit Wirtszellen nach Anspruch 10 oder 11, die ein Protein mit Epoxidhydrolaseaktivität nach einem der Ansprüche 1 bis 6 exprimieren, wobei erhalten wird:
- ein Gemisch der obigen Verbindungen (II) und (III), wobei die Verbindungen (II) und (III) gegebenenfalls durch einen zusätzlichen Reinigungsschritt voneinander getrennt werden können,
- oder nur die obige Verbindung der Formel (III).

15. Verfahren zur Herstellung eines rekombinanten Proteins mit Epoxidhydrolaseaktivität nach Anspruch 5 oder 6, **dadurch gekennzeichnet, dass** es einen Transformationsschritt von Wirtszellen, ausgewählt aus Bakterien, Viren, Hefen, Pilzen, Pflanzen oder Säugerzellen mit einem Vektor nach Anspruch 9 umfasst sowie einen Schritt der Aufreinigung der durch diese Zellen gebildeten rekombinanten Epoxidhydrolase.

16. Verfahren zur Herstellung eines zur Homogenität elektrophoretisch aufgereinigten Proteins mit Epoxidhydrolaseaktivität nach Anspruch 3 oder 4, wobei das Verfahren umfasst:
- einen Schritt der Extraktion des Enzyms aus Pilzzellkulturen, wie etwa Pilzen der Gattung *Aspergillus,* gefolgt von einem Schritt der Zentrifugation bei geringer Geschwindigkeit, der Wiedergewinnung des Überstands und gegebenenfalls der Einengung,
- einen Schritt der Aufreinigung des Enzyms aus dem im vorigen Schritt erhaltenen Extrakt durch aufeinander folgenden Durchlauf über Säulen von DEAE-Sepharose, Phenylsepharose, Mono Q und Superose 12.
